# EUROPÄISCHE PATENTSCHRIFT

(11) **EP 1 234 547 B1**
(45) Veröffentlichungstag und Bekanntmachung des Hinweises auf die Patenterteilung: **10.03.2004**
(21) Anmeldenummer: 01125465.3
(22) Anmeldetag: 06.11.2001
(51) Int. Cl.: A61B 17/70

(54) **Knochenschraube**
Bone screw
Vis pour l'ancrage osseux

(30) Priorität: 17.02.2001 DE 10108965
(43) Veröffentlichungstag der Anmeldung: 28.08.2002
(73) Patentinhaber: Schäfer, Bernd, 6315 Oberägeri (CH)
(72) Erfinder: Schäfer, Bernd, 6315 Oberägeri (CH); Halm, Henry, Dr., 23730 Neustadt (DE)
(74) Vertreter: Dreiss, Fuhlendorf, Steimle & Becker

(56) Entgegenhaltungen:
- EP-A- 0 535 623
- EP-A- 1 064 885
- EP-A- 1 190 678
- US-A- 5 346 493

## Beschreibung

Die Erfindung betrifft eine Knochenschraube, insbesondere Pedikelschraube, mit den Merkmalen des Oberbegriffs des Anspruchs 1.

Aus der EP 0 699 055 B1 ist eine Osteosynthesevorrichtung bekannt, die eine Knochenschraube mit einem Gabelkopf aufweist, in welchen ein Korrekturstab eingelegt werden kann. Der Korrekturstab wird von einer Kopfmutter festgehalten, die auf ein an den Schenkeln des Gabelkopfes vorgesehenes Gewinde aufgeschraubt wird. Es hat sich gezeigt, dass Situationen existieren, bei welchen das Aufschrauben der Kopfmutter mit Schwierigkeiten verbunden ist oder wenn der Chirurg keinen direkten Blickkontakt zur Schraube besitzt ein Aufschrauben problematisch sein kann.

Aus der EP-A-1 064 885 ist eine Haltevorrichtung für die Wirbelsäule bekannt geworden, die eine Schraube mit einem Bajonettverschluss aufweist. Dieser Bajonettverschluss hat den wesentlichen Nachteil, dass er Teile, z.B.

Bajonettzapfen, aufweist, bei denen die Gefahr besteht, dass sie sich mit der Zeit lösen und sich von der Schraube trennen, weshalb sie mit großem Aufwand sicher an der Schraube befestigt werden müssen.

Der Erfindung liegt daher die Aufgabe zugrunde, eine Knochenschraube der eingangs genannten Art derart weiterzubilden, dass auch in schwierigen Situationen die Mutter einfach mit dem Gabelkopf verbunden werden kann.

Diese Aufgabe wird bei einer Knochenschraube gelöst, die die Merkmale des Anspruchs 1 aufweist.

Bei der erfindungsgemäßen Knochenschraube wird die Mutter nach Art eines Bajonettverschlusses auf die freien Enden der beiden Schenkel des Gabelkopfes aufgesetzt und geringfügig verdreht. Dieser Bajonettverschluss hat den wesentlichen Vorteil, dass die Mutter nicht mehr in ein Gewinde eingefädelt werden muss, sondern sie muss lediglich auf das Ende des Gabelkopfes aufgesteckt werden. Dies kann in schwierigen Situationen und bei ungenügenden Sichtverhältnissen problemlos stattfinden.

Erfindungsgemäß ist vorgesehen, dass wenigstens ein freies Ende des Gabelkopfes mit einem axialen Fortsatz versehen ist. Über diesen axialen Fortsatz wird die maximale Verdrehung der Mutter und somit die Schließlage des Bajonettverschlusses geschaffen. Hierfür ist die Mutter an ihrer Innenfläche mit einer Aufnahmenut für den Fortsatz versehen, wobei sich die Aufnahmenut über einen Teil des Innenumfangs erstreckt. Beim Aufsetzen der Mutter auf die Enden des Gabelkopfes greift der Fortsatz in die Aufnahmenut ein und die Mutter kann so lange verdreht werden, bis der Fortsatz am Ende der Aufnahmenut angelangt ist.

Eine bevorzugte Weiterbildung sieht vor, dass die freien Enden radial nach außen abragende Vorsprünge aufweisen. Diese Vorsprünge erstrecken sich über die Umfangslänge der freien Enden des Gabelkopfes. Auf diese Weise können relativ große Kräfte übertragen werden und die Flächenpressungen sind relativ klein.

Erfindungsgemäß weist die Mutter am Innenumfang zwei radial nach innen vorspringende Nasen auf, die sich über jeweils 20° bis 120°, vorzugsweise 85° des Innenumfangs erstrecken. Hinter diese Nasen greifen die Vorsprünge der freien Enden des Gabelkopfes und bilden dadurch den Bajonettverschluss. Eine andere Ausführungsform sieht vor, dass die Mutter an ihrer Innenseite mit einem Anschlag für den Fortsatz versehen ist. Bei dieser Ausführungsform greift der Anschlag in den Weg des Fortsatzes ein, so dass die Mutter so lange verdreht werden kann, bis der Fortsatz am Anschlag anliegt.

Eine Weiterbildung sieht vor, dass bei auf den Gabelkopf aufgesetzter Mutter der Abstand zwischen dem Nutgrund des Gabelkopfes und dem der Nut zugewandten Seite der Mutter größer ist als die lichte Weite der Gabelschenkel des Gabelkopfes. Dies bedeutet, dass bei in den Gabelkopf eingelegtem Korrekturstab und auf die freien Enden des Gabelkopfes aufgesetzter Mutter die Mutter nicht auf dem Korrekturstab aufliegt. Sie kann daher mühelos gedreht und der Bajonettverschluss geschlossen werden.

Eine Weiterbildung sieht vor, dass die Mutter mit einer zentralen Gewindebohrung für eine Schraube, insbesondere eine Madenschraube, versehen ist. Die Fixierung des Korrekturstabes im Gabelkopf erfolgt über diese Madenschraube, die nach dem Schließen des Bajonettverschlusses in die Mutter eingeschraubt und auf den Korrekturstab aufgeschraubt und dadurch aufgepresst wird. Vorzugsweise weist die Madenschraube an ihrem eingeschraubten Ende eine Spitze bzw. eine Ringschneide auf, die sich in die Oberfläche des Korrekturstabes eingräbt. Durch die Madenschraube wird sowohl der Korrekturstab im Grund des Gabelkopfes festgehalten, als auch die Mutter am Gabelkopf gesichert und der Bajonettverschluss in der Schließlage gehalten.

Vorzugsweise entspricht die Einschraubrichtung der Madenschraube der Drehrichtung des Bajonettverschlusses, so dass die Mutter beim Einschrauben der Madenschraube nicht festgehalten werden muss. Auch ein Lösen der Mutter kann dadurch relativ einfach erfolgen, da die Aufschraubrichtung für die Madenschraube der Drehrichtung des Bajonettverschlusses für das Lösen entspricht.

Zum Drehen der Mutter besitzt diese an ihrem Außenumfang eine Werkzeugangriffsfläche, insbesondere einen Sechskant. Auf diese Weise kann die Mutter in beide Drehrichtungen einfach bewegt und der Bajonettverschluss geschlossen und geöffnet werden.

Weitere Vorteile, Merkmale und Einzelheiten der Erfindung ergeben sich aus der nachfolgenden Beschreibung, in der unter Bezugnahme auf die Zeichnung ein besonders bevorzugtes Ausführungsbeispiel im Einzelnen beschrieben ist. Dabei können die in der Zeichnung dargestellten und in den Ansprüchen sowie in der Beschreibung erwähnten Merkmale jeweils einzeln für sich oder in beliebiger Kombination erfindungswesentlich sein. In der Zeichnung zeigen:
- Figur 1: eine perspektivische Ansicht der Knochenschraube mit eingesetztem Korrekturstab;
- Figur 2: eine perspektivische Ansicht der Schraube bei abgenommener Mutter; und
- Figur 3: eine perspektivische Ansicht der Mutter.

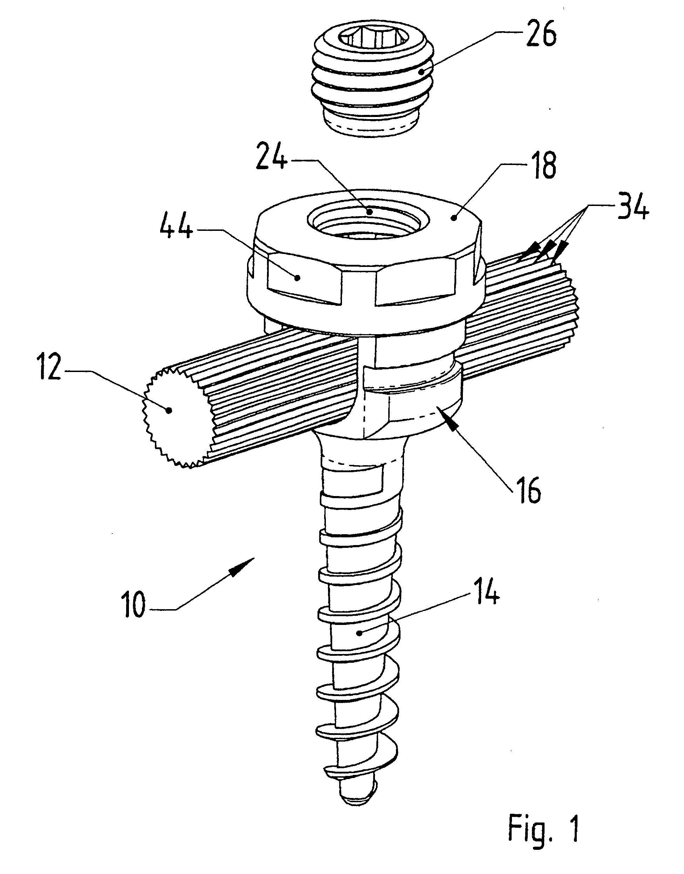

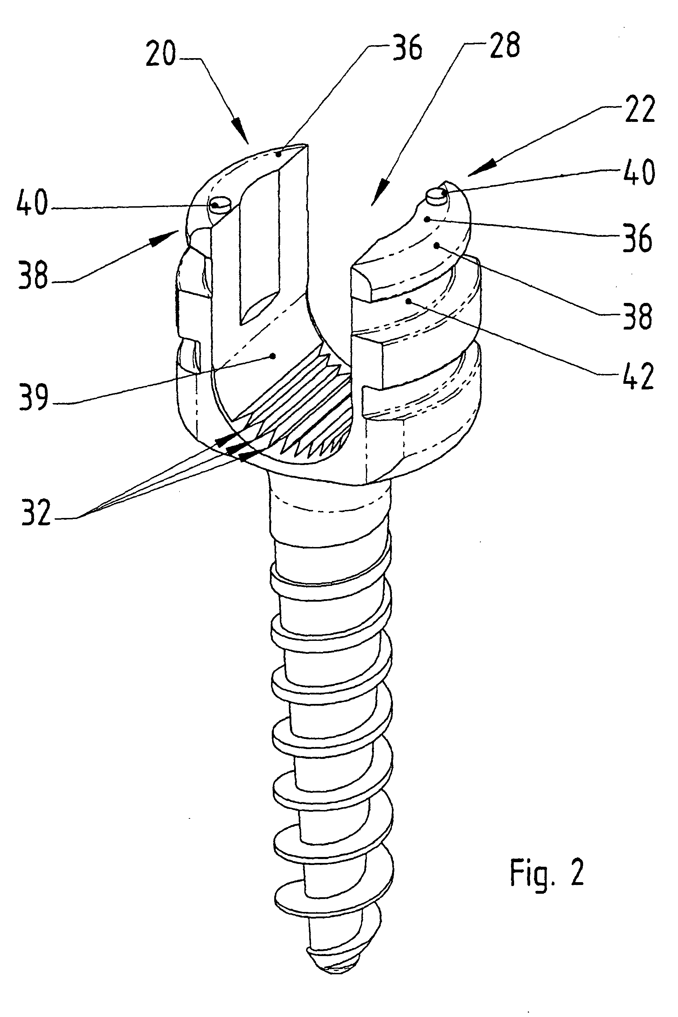

Die Figur 1 zeigt ein besonders bevorzugtes Ausführungsbeispiel gemäß Erfindung, wobei mit dem Bezugszeichen 10 eine Knochenschraube bezeichnet ist. Mit dieser Knochenschraube 10 ist ein Korrekturstab 12 verbunden. Zum Einschrauben der Knochenschraube 10 in einen Knochen, einen Wirbel oder dergl. weist diese einen Schaft 14 auf, der mit einem Gewinde versehen ist. Der Schaft 14 ist konisch ausgebildet und geht an seinem oberen Ende in einen Gabelkopf 16 über, in welchem der Korrekturstab 12 gelagert ist. Auf dem Gabelkopf 16 befindet sich eine Mutter 18, die den Gabelkopf 16 teilweise umgreift und verschließt und die beiden Schenkel 20 und 22 des Gabelkopfes 16 (siehe Figur 2) gegen Auseinanderdrücken sichert. Die Oberseite der Mutter 18 ist mit einer zentralen Gewindebohrung 24 versehen, in welche eine Madenschraube 26 einschraubbar ist, wodurch der Korrekturstab 12 sicher im Gabelkopf 16 fixiert wird.

In der Figur 2 ist die Nut 28 des Gabelkopfes 16 deutlich erkennbar und sind am Grund 30 der Nut 28 mehrere in Längsrichtung der Nut 28 verlaufende Kerben 32 dargestellt. Diese Kerben 32 greifen in Längsnuten 34 des Korrekturstabes 12 ein und bilden dadurch eine Verdrehsicherung für den Korrekturstab 12. Die freien Enden 36 der beiden Schenkel 20 und 22 sind mit radial nach außen abragenden Vorsprüngen 38 versehen, die einen Teil eines Bajonettverschlusses bilden. Auf den Vorsprüngen 38 sind an diametral gegenüberliegenden Seiten zwei Fortsätze 40 vorgesehen, die axial abragen. Unterhalb der Vorsprünge 38 befindet sich jeweils eine Nut 42, die sich, ebenso wie die Vorsprünge 38, über die gesamte Umfangsbreite der Schenkel 20 und 22 erstreckt.

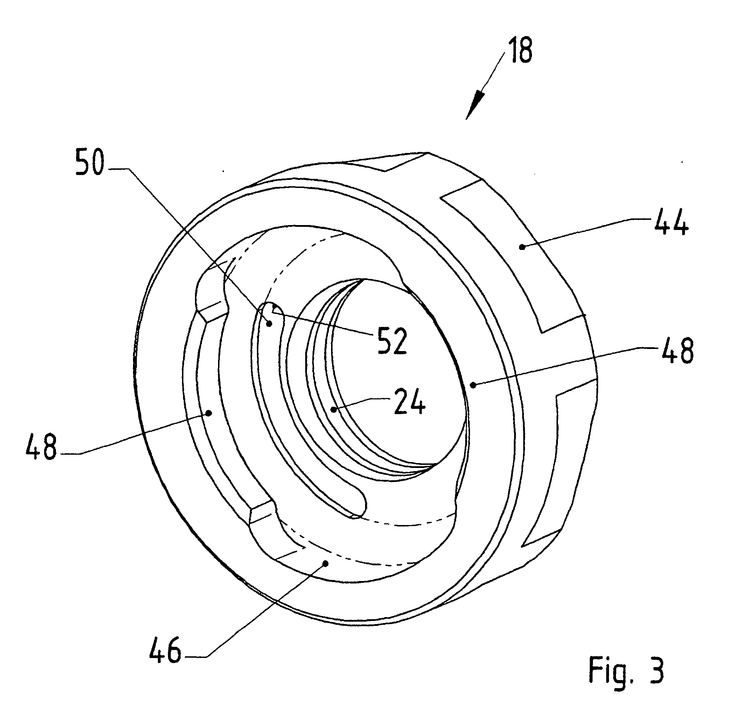

Die in der Figur 3 dargestellte Mutter 18 besitzt an ihrem Außenumfang eine Werkzeugangriffsfläche 44, welche als Sechskant ausgebildet ist. Die Innenseite 46 der Mutter 18 ist mit zwei einander diametral gegenüberliegenden radial nach innen vorspringenden Nasen 48 versehen. Diese Nasen 48 erstrecken sich über etwa 85° des Innenumfangs. Bei auf den Gabelkopf 16 aufgesetzter Mutter 18, wie in der Figur 1 dargestellt, greifen diese Nasen 48 in die hierfür vorgesehene Nuten 42 unterhalb der Vorsprünge 38 und bilden dadurch den Bajonettverschluss. Eine Drehbegrenzung der Mutter 18 wird über die Fortsätze 40 erreicht, die bei auf den Gabelkopf 16 aufgesetzter Mutter 18 in eine Aufnahmenut 50 eingreifen, die ebenfalls an der Innenseite 46 der Mutter 18 vorgesehen ist. Beim axialen Aufsetzen der Mutter 18 auf den Gabelkopf 16 greifen die Fortsätze 40 in diese Aufnahmenut 50 ein und erlauben das Verdrehen der Mutter 18 lediglich in eine Drehrichtung, bis die Fortsätze 40 an der Nutwand 52 anliegen. Hierdurch wird sichergestellt, dass die Mutter 18 und auch der Bajonettverschluss lediglich in einer Drehrichtung geschlossen werden kann, nämlich in mathematisch positiver Drehrichtung. Wird dann die Madenschraube 26 in die Gewindebohrung 24 der Mutter 18 eingeschraubt, dann wird die Mutter 18 in dieser Schließposition gehalten und es bedarf keiner Unterstützung von außen.

## Patentansprüche

1. Knochenschraube, insbesondere Pedikelschraube, mit einem eine Nut aufweisenden Gabelkopf (16) zur Aufnahme eines Korrekturstabes (12), mit einem ein Gewinde aufweisenden Schraubenschaft (14) und mit einer Mutter (18) für die freien Enden (36) des Gabelkopfes (16), wobei die freien Enden (36) des Gabelkopfes (16) und die Mutter (18) einen Bajonettverschluss bilden, **dadurch gekennzeichnet, dass** wenigstens ein freies Ende (36) des Gabelkopfes (16) mit einem axialen Fortsatz (40) versehen ist und dass die Mutter (18) an ihrer Innenseite (46) mit einer Aufnahmenut (50) für den Fortsatz (40) versehen ist und die Aufnahmenut (50) sich über einen Teil des Innenumfangs erstreckt.

2. Knochenschraube nach Anspruch 1, **dadurch gekennzeichnet, dass** die freien Enden (36) radial nach außen abragende Vorsprünge (38) aufweisen.

3. Knochenschraube nach Anspruch 2, **dadurch gekennzeichnet, dass** die Vorsprünge (38) sich über die Umfangslänge der freien Enden (36) erstrecken.

4. Knochenschraube nach einem der vorhergehenden Ansprüche, **dadurch gekennzeichnet, dass** die Mutter (18) am Innenumfang zwei radial nach innen vorspringende Nasen (48) aufweist.

5. Knochenschraube nach Anspruch 4, **dadurch gekennzeichnet, dass** sich die Nasen (48) über jeweils 20° bis 90° des Innenumfangs erstrecken.

6. Knochenschraube nach einem der vorhergehenden Ansprüche, **dadurch gekennzeichnet, dass** die Mutter (18) an ihrer Innenseite (46) mit einem Anschlag (52) für den Fortsatz (40) versehen ist.

7. Knochenschraube nach einem der vorhergehenden Ansprüche, **dadurch gekennzeichnet, dass** bei auf den Gabelkopf (16) aufgesetzter Mutter (18) der Abstand zwischen dem Nutgrund (30) des Gabelkopfes (16) und dem der Nut zugewandten Ende der Mutter (18) größer ist als die lichte Weite der Schenkel (20, 22) des Gabelkopfes (16).

8. Knochenschraube nach einem der vorhergehenden Ansprüche, **dadurch gekennzeichnet, dass** die Mutter (18) mit einer zentralen Gewindebohrung (24) für eine Schraube, insbesondere eine Madenschraube (26), versehen ist.

9. Knochenschraube nach Anspruch 9, **dadurch gekennzeichnet, dass** die Einschraubrichtung der Madenschraube (26) der Schließrichtung des Bajonettverschlusses entspricht.

10. Knochenschraube nach einem der vorhergehenden Ansprüche, **dadurch gekennzeichnet, dass** die Mutter (18) an ihrem Außenumfang eine Werkzeugangriffsfläche (44), insbesondere einen Sechskant, aufweist.

## Claims

1. Bone screw, in particular, pedicle screw, comprising a forked head (16) with a groove for receiving a correction rod (12), and with a threaded screw shaft (14) as well as a nut (18) for the free ends (36) of the forked head (16), wherein the free ends (36) of the forked head (16) and the nut (18) form a bayonet catch, **characterized in that** at least one free end (36) of the forked head (16) is provided with an axial extension (40) and that the inner side (46) of the nut (18) comprises a receiving groove (50) for the extension (40), the receiving groove(50) extending over part of the inner periphery.

2. Bone screw according to claim 1, **characterized in that** the free ends (36) comprise radially outwardly protruding projections (38).

3. Bone screw according to claim 2, **characterized in that** the projections (38) extend over the peripheral length of the free ends (36).

4. Bone screw according to any one of the preceding claims, **characterized in that** the inner circumference of the nut (18) comprises two radially inwardly projecting noses (48).

5. Bone screw according to claim 4, **characterized in that** each nose (48) extends through 20° to 90° of the inner periphery.

6. Bone screw according to any one of the preceding claims, **characterized in that** the inner side (46) of the nut (18) comprises a stop (52) for the extension (40).

7. Bone screw according to any one of the preceding claims, **characterized in that** the distance between the groove bottom (30) of the forked head (16) and the end of the nut (18) facing the groove (30) is larger than the inside extent of the legs (20, 22) of the forked head (16) when the nut (18) is attached onto the forked head (16).

8. Bone screw according to any one of the preceding claims, **characterized in that** the nut (18) comprises a central threaded bore (24) for a screw, in particular a slug (26).

9. Bone screw according to claim 10, **characterized in that** the screwing-in direction of the slug (26) corresponds to the closing direction of the bayonet catch.

10. Bone screw according to any one of the preceding claims, **characterized in that** the outer circumference of the nut (18) comprises a tool engagement surface (44), in particular, a hexagon.

## Revendications

1. Vis pour l'ancrage osseux, en particulier vis pédiculaire, comprenant une tête fourchue (16) présentant une rainure pour recevoir une tige de correction (12), un corps de vis (14) présentant un filetage, et un écrou (18) destiné à coopérer avec les extrémités libres (36) de la tête fourchue (16), lesdites extrémités libres (36) de la tête fourchue (16) et ledit écrou (18) formant conjointement une fermeture à baïonnette, **caractérisée en ce qu'**au moins une extrémité libre (36) de la tête fourchue (16) présente un prolongement axial (40), **en ce que** l'écrou (18) est doté, sur sa face interne (46), d'une rainure de réception (50) du prolongement (40) et **en ce que** la rainure de réception (50) s'étend sur une partie de la périphérie interne.

2. Vis pour ancrage osseux selon la revendication 1, **caractérisée en ce que** les extrémités libres (36) présentent des ressauts (38) qui dépassent radialement vers l'extérieur.

3. Vis pour ancrage osseux selon la revendication 2, **caractérisée en ce que** les ressauts (38) s'étendent sur la longueur périphérique des extrémités libres (36).

4. Vis pour ancrage osseux selon l'une des. revendications précédentes, **caractérisée en ce que** l'écrou (18) présente, sur sa périphérie interne, deux nez (48) qui dépassent radialement vers l'intérieur.

5. Vis pour ancrage osseux selon la revendication 4, **caractérisée en ce que** les nez (48) s'étendent chacun sur 20° à 90° de la périphérie interne.

6. Vis pour ancrage osseux selon l'une des revendications précédentes, **caractérisée en ce que** l'écrou (18) présente, sur sa face interne (46), une butée (52) pour le prolongement (40).

7. Vis pour ancrage osseux selon l'une des revendications précédentes, **caractérisée en ce que** lorsque l'écrou (18) est rapporté sur la tête fourchue (16), la distance entre le fond de la rainure (30) de la tête fourchue (16) et l'extrémité de l'écrou (18) en regard de la rainure est supérieure à l'ouverture des doigts (20, 22) de la tête fourchue (16).

8. Vis pour ancrage osseux selon l'une des revendications précédentes, **caractérisée en ce que** l'écrou (18) présente un taraudage central (24) destiné à recevoir une vis, en particulier une vis sans tête (26).

9. Vis pour ancrage osseux selon la revendication 9, **caractérisée en ce que** le sens de vissage de la vis sans tête (26) coïncide avec le sens de fermeture de la fermeture à baïonnette.

10. Vis pour ancrage osseux selon l'une des revendications précédentes, **caractérisée en ce que** l'écrou (18) présente, sur sa périphérie externe, une surface d'application d'un instrument (44), en particulier un six pans.
